# EUROPEAN PATENT SPECIFICATION

(11) **EP 0 254 596 B1**
(45) Date of publication and mention of the grant of the patent: **10.02.1993**
(21) Application number: 87306607.0
(22) Date of filing: 27.07.1987
(51) Int. Cl.: C07C 51/15, C07C 65/11

(54) **Manufacturing method for 2-hydroxynaphtalene-6-carboxylic acid**
Herstellungsverfahren für 2-Hydroxynaphthol-6-Carbonsäure
Procédé pour la fabrication de l'acide 2-hydroxynaphtalène-6-carboxylique

(30) Priority: 25.07.1986 JP 176254/86
(43) Date of publication of application: 27.01.1988
(73) Proprietor: Kabushiki Kaisha Ueno Seiyaku Oyo Kenkyujo, Higashi-ku Osaka-shi Osaka-fu (JP)
(72) Inventor: Ueno, Ryuzo, Nishinomiya-shi Hyogo-ken (JP); Masada, Yoshiyasu, Hirakatashi Osaka-fu (JP); Mori, Toru, Takarazuka-shi Hyogo-ken (JP)
(74) Representative: Atkinson, Peter Birch

(56) References cited:
- EP-A- 0 053 824
- EP-A- 0 081 753
- WO-A-81/02573
- US-A- 4 345 094
- US-A- 4 345 095

## Description

The present invention relates to an improved method of manufacturing 2-hydroxynaphthalene-6-carboxylic acid by the reaction of potassium β-naphtholate and carbon dioxide.

2-hydroxynaphthalene-6-carboxylic acid is an important raw material for every kind of aromatic polyester and is indispensable especially to the production of a liquid crystal polymer that has good workability and flowability, and a resin or fiber that has good elasticity and heat resistance.

There are many known methods of production of 2-hydroxynaphthalene-6-carboxylic acid, for example, as disclosed in U.S. Patent No. 1593816 (1926), and in Japanese patent KOKAI Nos. 95939/1982 and 212139/1982.

By such known methods, however, it had not been possible to produce 2-hydroxynaphthalene-6-carboxylic acid at a high yield and at a high selectivity. The term "selectivity" in the present specification means the ratio of 2-hydroxynaphthalene-6-carboxylic acid to (2-hydroxynaphthalene-6-carboxylic acid plus 2-hydroxynaphthalene-3-carboxylic acid).

In our early research to establish a method of producing 2-hydroxynaphthalene-6-carboxylic acid from β-naphthol on an industrial scale, it had been found that the product was obtainable in relatively good yields and at high selectivity when the reaction was allowed to proceed by selecting a carbon dioxide pressure suitable for the reaction temperature, and removing free β-naphthol from the reaction system by means of an overflow (reference: Japanese Patent Publication No. 35911/1984). It is understood that, in the above-mentioned Kolbe-Schmitt reaction, the intermediate formed by bonding of a potassium atom to the hydroxyl group of β-naphthol decomposes in the presence of water to return to β-naphthol. Therefore, it had been generally considered that this reaction must be carried out by first reacting β-naphthol with a potassium source in an aqueous solution, secondly dehydrating the potassium β-naphtholate obtained therefrom, and thirdly reacting with carbon dioxide. The degree of this dehydration has been considered to influence the yield and selectivity of the product.

According to the present invention there is provided a process for the preparation of 2-hydroxynaphthalene-6-carboxylic acid by reacting substantially anhydrous potassium β-naphtholate with carbon dioxide in a reaction vessel and in the presence of a reaction medium that does not substantially dissolve the β-naphtholate to give 2-hydroxynaphthalene-6-carboxylic acid, the improvement comprising maintaining the reaction temperature and pressure within the reaction vessel whereby β-naphthol and water in the vapour phase are removed from the reaction system.

It has been found that the yield and selectivity of 2-hydroxynaphthalene-6-carboxylic acid are adversely influenced by free β-naphthol, 2-hydroxynaphthalene-3-carboxylic acid produced during the reaction, water resulting from the potassium source in the reaction system (for example, see the reaction schemes (i) - (v) below) and the residual water in potassium β-naphtholate. It has also been found that the yield and selectivity can be improved, and the side reactions, such as the formation of β-naphthol dimer, condensates of β-naphthol with naphthoic acid, tar and the like can be decreased to give the 2-hydroxynaphthalene-6-carboxylic acid in a higher yield and with an excellent selective ratio, if the water is removed together with the β-naphthol from the reaction system during the reaction.

Further it has been found that if the elimination is achieved by transferring the water and the free β-naphthol, together with carbon dioxide gas from the reaction system, to the vapour phase, (and not by overflowing as in the conventional method), the yield and selectivity can be improved remarkably, and the side reactions can be effectively prevented. 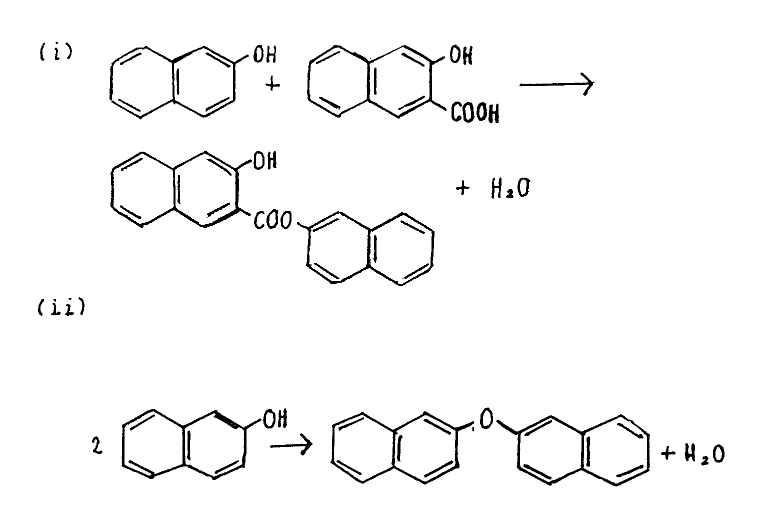 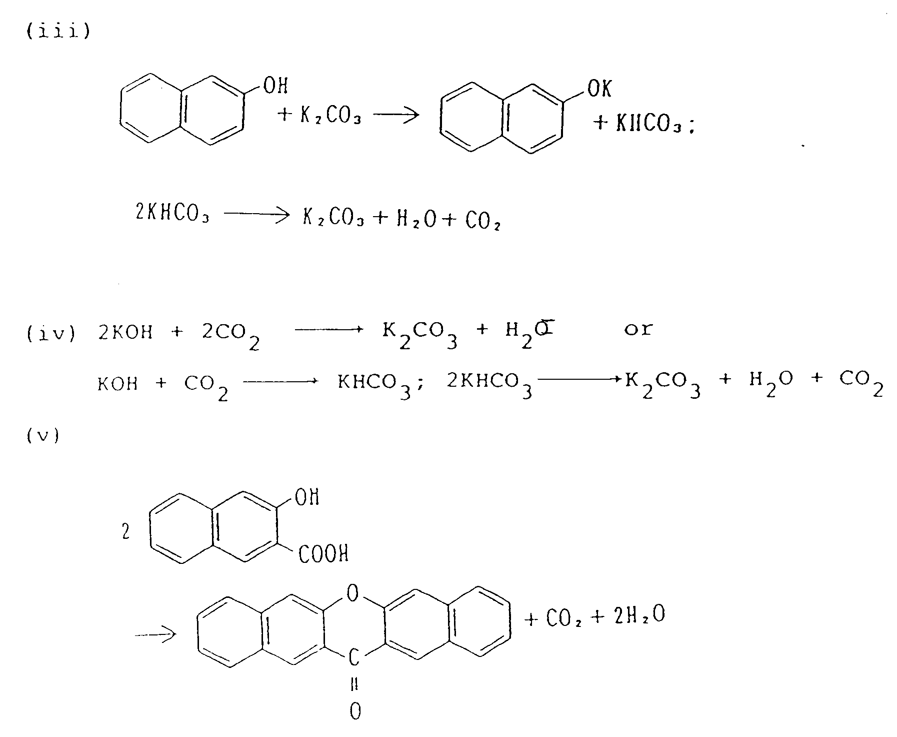
The method according to the present invention is superior to the conventional method in which the β-naphthol is eliminated from the reaction system (to prevent the side reaction) by the overflow.

In the present invention the yield and selectivity of the 2-hydroxynaphthalene-6-carboxylic acid can be improved.

According to the present invention, potassium β-naphtholate and carbon dioxide are reacted in the presence of a reaction medium that does not substantially dissolve the potassium β-naphtholate under the reaction conditions.

The potassium β-naphtholate for this reaction can be prepared from β-naphthol and an alkaline potassium compound by a usual method and, when used, the product is required to be sufficiently dehydrated. For the formation of the potassium β-naphtholate it is preferable to employ an alkaline potassium compound such as potassium hydroxide or potassium carbonate. β-Naphthol is to be employed in a quantity within the range of 0.97-1.03 mol (preferably in the vicinity of 1.00 mol), against one equivalent of an alkaline potassium compound.

The β-naphthol reacts easily with an alkaline potassium compound, for instance, by addition of β-naphthol into an aqueous solution of potassium hydroxide to give a concentrated solution of potassium β-naphtholate. The potassium β-naphtholate is dehydrated, for example, by heating at a temperature of 240°C or higher, (preferably in the range of 250-300°C), in a stream of an inert gas such as nitrogen either under atmospheric pressure or an elevated or reduced pressure. Since the melting point of potassium β-naphtholate is about 235°C, it is in a liquid state at the above-mentioned heating temperature range and is therefore capable of being dehydrated continuously or batchwise without a reaction medium. Such dehydration without reaction medium is advantageous because it does not need a large apparatus nor a powerful and energetic stirring operation that would otherwise be required to evenly mix the potassium β-naphtholate with the medium due to their large differences in specific gravity. Also, from the viewpoint of improving the yield and selectivity of the product, a medium for the dehydration is preferably not used. This is because the reaction between β-naphthol and carbon dioxide may be conducted with a reaction medium at an accurate ratio and at a suitable temperature. Thus the heat evolved from the reaction of potassium β-naphtholate with carbon dioxide can be easily eliminated in such a manner as to achieve a satisfactory adjustment of the reaction temperature for improvement of said yield and selectivity.

The reaction between potassium β-naphtholate and carbon dioxide may be carried out at a reaction temperature within a range of 230-350°C (preferably within a range of 240-320°C), and under a pressure of carbon dioxide at a suitable value corresponding to the reaction temperature, but within a range of 1-20 kg/cm² (1-20 x 10³ Pa) (G), preferably within a range of 2-16 kg/cm² (2-16 x 10³ Pa) (G). The pressure of carbon dioxide is selected preferably within a range of 2-7 kg/cm² (2-7 x 10³ Pa) (G) for a reaction temperature of 260°C and within a range of 2-10 kg/cm² (2-10 x 10³ Pa) (G) for 280°C.

In the most preferred embodiment carbon dioxide is dispersed into the reaction mixture in such a manner that carbon dioxide is blown through a blow nozzle with stirring. By such sparging of the carbon dioxide, the carboxylation of β-naphthol can be accelerated; the elimination of free β-naphthol from the reaction system can be effectively achieved, and side reactions such as the formation of β-naphthol dimer, tar and the like can be reduced in comparison with the overflow method. Therefore, the sparging of the carbon dioxide into the reaction mixture is extremely effective to improve the yield and selectivity of 2-hydroxynaphthalene-6-carboxylic acid.

Applicable in the practice of this invention is a reaction medium which does not dissolve potassium β-naphtholate to any substantial degree and whose specific gravity at ambient temperature ranges from 0.6 to 1.5, preferably from 0.7 to 1.4. The reaction medium suitable for the present invention can be selected from aliphatic, alicyclic or aromatic hydrocarbons or ethers thereof, for example, light oil, kerosene, gasoline, lubricating oil, alkylbenzenes, alkylnaphthalenes, diphenyls, diphenylalkanes, alkyldiphenyls, triphenyls, hydrogenated triphenyls, diphenyl ethers, alkylphenyl ethers, alkyldiphenyl ethers, etc., and mixtures thereof. For the present invention such reaction media having a boiling point within the range of 150-400°C, especially a range of 180-400°C, are preferable.

The reaction medium is employed in a quantity which is at least 0.5 times by weight that of potassium β-naphtholate, preferably within a range of 0.5-10 times and most preferably within the range of 1-5 times. When such a reaction medium is employed for the process of forming potassium β-naphtholate and/or for its dehydration, it is preferable to use the reaction medium in a quantity including an additional portion to form an azeotrope with water.

A method embodying the present invention, ie the reaction of potassium β-naphtholate with carbon dioxide in the presence of a reaction medium that does not substantially dissolve potassium β-naphtholate under the reaction conditions, consists essentially of stripping by-produced β-naphthol together with the water existing in the reaction mixture to the vapour phase, and removing them from the reaction system quickly under a flow of carbon dioxide.

In the practice of the prior manufacturing method also developed by the present inventors (reference: Japanese Patent Publication No. 35911/1984) wherein β-naphthol is removed from the reaction system by an overflow together with the reaction medium, such as light oil, it is very difficult to remove the water existing in the reaction system efficiently.

The method for the transference of β-naphthol and water to the vapour phase and their removal from the reaction system in the practice of this invention my be effected by the following illustrative (but non-limiting) techniques:
(1) the gas in the reaction vessel is intermittently discharged throughout the reaction period and pressurized carbon dioxide is introduced as a replenishment;
(2) the gas in the reaction vessel (ie. carbon dioxide, β-naphthol and water) is continuously discharged therefrom while pressurized carbon dioxide is continuously sparged into the reaction mixture so as not to allow the pressure to fall;
(3) the gas in the reaction vessel is cooled by a heat exchanger set in the line connected to the reaction vessel so that the by-produced β-naphthol and water is removed from the reaction system as a liquid condensate continuously or intermittently. Carbon dioxide is recycled to the reaction vessel.

In order to transfer the β-naphthol and the water into the vapour phase and remove them from the reaction system efficiently and quickly, it is better to carry out the reaction of potassium β-naphtholate with carbon dioxide under sufficient stirring in the presence of the reaction medium which (i) can disperse but does not dissolve the potassium β-naphtholate at the reaction temperature of about 250 - 300°C, (ii) does not have a vapour pressure so high as to remarkably increase the reaction pressure, and (iii) has no substantial influence on the reaction.

This reaction can be carried out batchwise or in a continuous procedure.

In the above-described reaction of potassium β-naphtholate with carbon dioxide, it is advantageous to add about 1-1.5 equivalent of potassium source based on the free β-naphthol by-produced in the reaction to the reaction system. Examples of the potassium source which may be used include potassium carbonate, potassium bicarbonate, alkylpotassium carbonate, alkoxypotassium, alkylpotassium, and potassium sulfate. For example, the existence of such a source of potassium in a quantity approximately within a range of 1-1.5 mol of free β-naphthol can reconvert the β-naphthol to potassium β-naptholate which can react with carbon dioxide, and, moreover, as the free β-naphthol can be reduced by the reconversion, the water which stems from the β-naphthol dimerisation is also reduced, so that the yield of the product can be improved.

The finishing process, for example, can be carried out as follows. After the reaction with carbon dioxide, water is added to the mixture and its pH value is adjusted to 6.5-8 with an acid, such as sulfuric acid or hydrochloric acid, to liberate β-naphthol from potassium β-naptholate in the reaction mixture. Before or after the above-mentioned step, the layer of reaction medium is separated and, if necessary, the mixture of β-naphthol and tarry substances liberated in the aqueous layer is settled and separated. The β-naphthol and tar layer that has been separated out is washed with water and the washing water is returned to the separated aqueous layer. Said aqueous layer is extracted with a hydrophobic solvent at a temperature of 110°C or lower. Applicable to this extraction are such solvents as hydrocarbons, halogenated hydrocarbons, nitrated hydrocarbons, ethers, ketones, and alcohols having a carbon atom number of four or more. Such a solvent for extraction is used in an amount of 0.3-2 times the volume of the aqueous layer and at a temperature of 30-110°C. The β-naphthol in the layer of the reaction medium may be recycled directly without further treatments, or preferably used by collecting it in an aqueous solution of potassium β-naphtholate by reacting aqueous potassium hydroxide with the β-naphthol contained in both the layer of the reaction medium and the extracting solvent. The β-naphthol in the extracting solvent layer or the tar layer can also be recovered by reduced pressure distillation. The aqueous solution of potassium β-naptholate and the β-naphthol thus recovered may be recycled in the preparation of raw materials.

In a procedure for recovering the product, the aqueous layer thus extracted is adjusted to the pH value of about 3-5, preferably about 3.5-4.5, to give 2-hydroxynaphthalene-6-carboxylic acid with high purity. If the pH of the aqueous layer is adjusted to a value of about 1-3, preferably to a value of about 1.5-2.5, a mixture of 2-hydroxynaphthalene-6-carboxylic acid and 2-hydroxynaphthalene-3-carboxylic acid is obtained. These two acids can easily be fractionated by, for example, treating with an organic solvent or a mixture of an organic solvent with water.

According to the intended use of the product, said extracted water layer may be subjected directly to acid precipitation at a pH value below 3. The improvement of the yield and selectivity of 2-hydroxynaphthalene-6-carboxylic acid in the present invention is achieved by the aforementioned simplified acid precipitation.

In the reaction between potassium β-naphtholate and carbon dioxide of the present invention, the β-naphthol which is formed during the reaction and plays a role in the various side reactions and water whose existence in the reaction system causes the low yield of the product are quickly transferred in vapour, cooled in a heat exchanger and removed as liquid condensate from the reaction system so that the reaction brings about improvement not only in the yield but also in the selectivity of 2-hydroxynaphthalene-6-carboxylic acid.

The present invention is illustrated by the following Examples, but is not restricted thereto,

### Example 1

Into a one-litre autoclave were charged 252g of 70% aqueous solution of potassium β-naphtholate, 26.5g of 50% aqueous solution of potassium carbonate, and 365g of light oil, and the mixture was subjected to dehydration at 260°C for 3 hours with stirring under inert gas atmosphere. The vaporised light oil was condensed and returned to the mixture. Pressure was applied to the inside of the autoclave so as to control the CO₂ gas pressure at 3 kg/cm² (3 x 10³ Pa) (G). The reaction was continued at 260°C for about 6 hours, and the CO₂ was sparged in the reaction mixture through a blow nozzle under vigorous agitation. The CO₂ gas was discharged from an outlet nozzle at the rate of 72 liter/hr, and along with it the β-naphthol and water in a gaseous form were discharged, cooled and removed from the autoclave during the reaction.

After conclusion of the reaction, the mixture in the autoclave was cooled and water was added. After the layer of light oil was separated, the water layer was subjected to acid precipitation to give 2-hydroxynaphthalene-6-carboxylic acid.

The yield and selectivity of the carboxylic acid are shown in Table 1.

### EXAMPLE 2

The reaction was conducted in a one-liter autoclave, having a heat exchanger (300 cm³) attached thereto, under conditions similar to those in Example 1.

The condensates were removed from the reaction system through an outlet in the lower part of the heat exchanger and the uncondensed CO₂ gas was returned from the top of the heat exchanger through a blow nozzle to the reaction mixture.

The yield and selectivity of 2-hydroxynaphthalene-6-carboxylic acid obtained are shown in Table 1.

### EXAMPLE 3

In a 50-liter autoclave were charged 8.7 kg of β-naphthol, 22 kg of light oil, 7 kg of 48% potassium hydroxide and 0.9 kg of potassium carbonate. The mixture was heated at 260°C for 5 hours with stirring to provide dehydrated potassium β-naphtholate. The light oil condensate was removed of water and returned to the reaction system.

Next, the reaction temperature was raised to 265°C, and while the gases in the reaction system were discharged at the rate of 4 Nm³/hr and condensed in a heat exchanger (part of the light oil contained in the liquid condensate was returned to the reaction system after drying), the reaction system was supplemented with CO₂ gas so that the gas pressure was maintained at 3.0 kg/cm² (3 x 10³ Pa) (G).

After the reaction was continued for 6 hours, the reaction mixture was cooled to 100°C, and then water was added to form two layers. Unreacted β-naphthol was recovered from the oil layer and the water layer was adjusted to a pH value of 3.5 to precipitate 2-hydroxynaphthalene-6-carboxylic acid (the yield and selectivity are shown in Table 1).

### EXAMPLE 4

Two 50 liter autoclaves, No.1 and No.2, were connected to each other, and potassium β-naphtholate dispersed in light oil prepared in the same manner as in Example 3 was supplied to No.1 autoclave at a rate of 8 kg/hr. The temperature of both of the two autoclaves was adjusted to 269°C and their CO₂ gas pressure was adjusted to 2.9 kg/cm² (2.9 x 10³ Pa) (G), and the contents were stirred so that the potassium β-naphtholate including K₂CO₃, would not settle. The reaction mixture was transferred from No.1 autoclave into No.2 autoclave through the connection pipe. In the autoclaves CO₂ gas was sparged from the bottoms and was circulated at 2.9 kg/cm² (2.9 x 10³ Pa) of CO₂ pressure after condensation of β-naphthol, water and light oil in the heat exchanger. The reaction volumes of both autoclaves were maintained at a constant level with a balance of the feed rate to No.1 autoclave and the drain rate from No.2 autoclave.

The reaction mixture in No.2 autoclave was continuously drained at the rate of 8 kg/hr. By the same procedure as in Example 3, 2-hydroxynaphthalene-6-carboxylic acid was obtained (the yield and selectivity are shown in Table 1).

### EXAMPLES 5-8

Except that different reaction temperatures of 255°C (Example 5), 265°C (Example 6), 270°C (Example 7) and 280°C (Example 8) were employed, the same procedure as described in Example 2 was carried out to give 2-hydroxynaphthalene-6-carboxylic acid in the yields and selectivities as shown in Table 1.

### EXAMPLES 9-11

Except that the reaction pressures of 2 kg/cm² (2 x 10³ Pa) (G) (Example 9), 8 kg/cm² (8 x 10³ Pa) (G) (Example 10), and 4 kg/cm² (4 x 10³ Pa) (G) (Example 11), were applied, with the reaction temperature held at 270°, the same procedure described in Example 2 was carried out to give 2-hydroxynaphthalene-6-carboxylic acid in the yields and selectivities as shown in Table 1.

### EXAMPLES 12 and 13

The same procedure as described in Example 2 was carried out except that in Example 12 the reaction was continued for 6 hours in a quantity of light oil 3 times that of potassium β-naptholate with a molar ratio of potassium carbonate/potassium β-naphtholate being held at 1.3, and that in Example 13, the reaction was continued for 8 hours in a quantity of light oil 3 times that of potassium β-naptholate, with a molar ratio of potassium carbonate/potassium β-naphtholate being held at 1.3. 2-Hydroxynaphthalene-6-carboxylic acid was obtained at the yields and selectivities shown in Table 1.

### COMPARATIVE EXAMPLE 1

From 84 grams of β-naphthol potassium β-naphtholate was prepared by an ordinary method and dehydrated in a mixture of 1- and 2-isopropylnaphthalene as a medium. The 1- and 2-isopropylnaphthalene obtained as condensate from the dehydration were returned to the dehydrated potassium β-naphtholate.

This mixture was put in an autoclave and allowed to undergo a reaction at a temperature of 265°, under a CO₂ gas pressure of 3 kg/cm² (3 x 10³ Pa) (G), for 16 hours. 2-Hydroxynaphthalene-6-carboxylic acid was obtained in the yield and selectivity as shown in Table 1.

### COMPARATIVE EXAMPLE 2

β-Naphthol was treated with a 48% potassium hydroxide (14.4 kg/hr), and the potassium β-naphtholate obtained was dispersed in light oil (18.2 kg/hr) and allowed to undergo a reaction under the same conditions as described in Example 4 (no heat exchanger was attached; β-naphthol, moved along with the overflow of the light oil, was removed from the reaction system through No.2 autoclave).

2-Hydroxynaphthalene-6-carboxylic acid was obtained in the yield and selectivity as shown in Table 1.

### COMPARATIVE EXAMPLE 3

The reaction in the autoclave was carried out in the same manner as described in Example 1, except that CO₂ gas was not discharged from the autoclave during the reaction, and β-naphthol and water were not removed.

**TABLE 1**

| | | YIELD (%) | SELECTIVITY (%)^{(*)} |
|---|---|---|---|
| EXAMPLE | 1 | 52 | 98.0 |
| | 2 | 51 | 99 |
| | 3 | 53 | 99.2 |
| | 4 | 56 | 99.5 |
| | 5 | 30 | 96 |
| | 6 | 51 | 99 |
| | 7 | 52 | 98.5 |
| | 8 | 51 | 97 |
| | 9 | 32 | 85 |
| | 10 | 40 | 96 |
| | 11 | 52 | 99 |
| | 12 | 56 | 99.5 |
| | 13 | 49 | 99.0 |
| COMPARATIVE EXAMPLE | 1 | 27 | 90 |
| | 2 | 43 | 94 |
| | 3 | 30 | 89.6 |

| | | | |
|---|---|---|---|
| (*) (2-hydroxynaphthalene-6-carboxylic acid) X 100/ [ (2-hydroxynaphthalene-6-carboxylic acid) + (2-hydroxynaphthalene-3-carboxylic acid)]. | | | |

## Claims

1. A process for the preparation of 2-hydroxynaphthalene-6-carboxylic acid by reacting substantially anhydrous potassium beta-naphtholate with carbon dioxide in a reaction vessel and in the presence of a reaction medium that does not substantially dissolve the beta-naphtholate to give 2-hydroxynaphthalene-6-carboxylic acid, the improvement comprising maintaining reaction conditions of temperature and pressure within the reaction vessel whereby beta-naphthol and water are removed from the reaction mixture in the vapour phase.

2. The process of Claim 1, wherein said carbon dioxide is continuously sparged into the reaction mixture.

3. The process of Claim 2, wherein said carbon dioxide is blown into the reaction mixture through a blow nozzle.

4. The process of Claim 2, wherein said carbon dioxide is continuously sparged into said reaction mixture and carbon dioxide, beta-naphthol and water are continuously discharged form said reaction vessel.

5. The process of Claim 4, wherein said discharged carbon dioxide, water and beta-naphthol are cooled and the water and beta-naphthol are separated from the carbon dioxide by condensation to recover carbon dioxide.

6. The process of Claim 5, wherein said recovered carbon dioxide is returned to the reaction vessel.

7. The process of Claim 6, wherein said recovered carbon dioxide is sparged into the reaction mixture through a blow nozzle.

## Patentansprüche

1. Verfahren zur Herstellung von 2-Hydroxynaphthalin-6-carbonsäure durch Umsetzung von im wesentlichen wasserfreiem Kalium-β-naphtholat mit Kohlendioxid in einem Reaktionsgefäß und in Gegenwart eines Reaktionsmediums, das das β-Naphtholat im wesentlichen nicht auflöst, unter Bildung von 2-Hydroxy-6-carbonsäure, dadurch **gekennzeichnet**, daß man solche Reaktionsbedingungen der Temperatur und des Drucks innerhalb des Reaktionsgefäßes aufrechterhält, daß β-Naphthol und Wasser aus dem Reaktionsmedium in der Dampfphase entfernt werden.

2. Verfahren nach Anspruch 1, dadurch **gekennzeichnet**, daß man das genannte Kohlendioxid kontinuierlich in das Reaktionsmedium eindispergiert.

3. Verfahren nach Anspruch 2, dadurch **gekennzeichnet**, daß man das genannte Kohlendioxid in das Reaktionsmedium durch eine Blasdüse einbläst.

4. Verfahren nach Anspruch 2, dadurch **gekennzeichnet**, daß man das genannte Kohlendioxid kontinuierlich in das genannte Reaktionsmedium eindispergiert, und daß man Kohlendioxid, β-Naphthol und Wasser kontinuierlich aus dem genannten Reaktionsgefäß austrägt.

5. Verfahren nach Anspruch 4, dadurch **gekennzeichnet**, daß man das genannte ausgetragene Kohlendioxid, Wasser und β-Naphthol abkühlt, und daß man das Wasser und das β-Naphthol von dem Kohlendioxid durch Kondensation abtrennt, wodurch wiedergewonnenes Kohlendioxid erhalten wird.

6. Verfahren nach Anspruch 5, dadurch **gekennzeichnet**, daß man das genannte wiedergewonnene Kohlendioxid in das Reaktionsgefäß zurückführt.

7. Verfahren nach Anspruch 6, dadurch **gekennzeichnet**, daß man das genannte wiedergewonnene Kohlendioxid in dem Reaktionsmedium durch eine Blasdüse dispergiert.

## Revendications

1. Procédé pour la préparation d'acide 2-hydroxynaphtalène-6-carboxylique par réaction de β-naphtolate de potassium pratiquement anhydre avec du dioxyde de carbone dans un récipient de réaction et en présence d'un milieu de réaction qui ne dissout pratiquement pas le β-naphtolate pour obtenir de l'acide 2-hydroxynaphtalène-6-carboxylique, dont l'amélioration consiste à maintenir des conditions de température et de pression à l'intérieur du récipient de réaction grâce auxquelles le β-naphtol et l'eau sont éliminés du mélange de réaction en phase vapeur.

2. Procédé selon la revendication 1, dans lequel on fait continuellement barboter le dioxyde de carbone dans le mélange de réaction.

3. Procédé selon la revendication 2, dans lequel on insufle le dioxyde de carbone dans le mélange de réaction par une buse de soufflage.

4. Procédé selon la revendication 2, dans lequel on fait continuellement barboter le dioxyde de carbone dans le mélange de réaction et on évacue continuellement le dioxyde de carbone, le β-naphtol et de l'eau du récipient de réaction.

5. Procédé selon la revendication 4, dans lequel on refroidit le dioxyde de carbone, l'eau et le β-naphtol évacués et on sépare l'eau et le β-naphtol du dioxyde de carbone par condensation pour récupérer le dioxyde de carbone.

6. Procédé selon la revendication 5, dans lequel on renvoie le dioxyde de carbone récupéré dans le récipient de réaction.

7. Procédé selon la revendication 6, dans lequel on fait barboter le dioxyde de carbone récupéré dans le mélange de réaction par une buse de soufflage.
